# EUROPÄISCHE PATENTSCHRIFT

(11) **EP 0 270 829 B1**
(45) Veröffentlichungstag und Bekanntmachung des Hinweises auf die Patenterteilung: **17.03.1993**
(21) Anmeldenummer: 87116093.3
(22) Anmeldetag: 02.11.1987
(51) Int. Cl.: A61N 1/08

(54) **Reizstromgerät**
Stimulator
Stimulateur

(30) Priorität: 13.11.1986 DE 3638683
(43) Veröffentlichungstag der Anmeldung: 15.06.1988
(73) Patentinhaber: SIEMENS AKTIENGESELLSCHAFT, 80333 München (DE)
(72) Erfinder: Herzog, Ludwig, D-6948 Waldmichelbach (DE); Knapp, Volker, D-6948 Waldmichelbach (DE)

(56) Entgegenhaltungen:
- EP-A- 0 026 479
- EP-A- 0 173 419
- DE-A- 2 843 724

## Beschreibung

Die Erfindung bezieht sich auf ein Reizstromgerät gemäß Oberbegriff des Patentanspruchs 1.

Bei Reizstromgeräten dieser Art ist eine mitlaufende Stromwertanzeige, die sich bei Stromarten mit längeren Pausen oder bei Schwellströmen offenbart, wünschenswert. Dies ist insbesondere auch wichtig bei Konstantspannungsbetrieb, da sich bekanntlich der Strom aufgrund sich ändernden Patientenwiderstandes unkontrolliert ändern kann. Das Gerät muß also fortlaufend den sich einstellenden Strom messen und anzeigen, so daß die Anzeige dem jeweiligen Momentanwert folgt. Bei einer mitlaufenden Stromanzeige ist es aber häufig nicht ganz einfach, den Spitzenstromwert anzuzeigen, insbesondere dann, wenn die eingesetzten Reizstromimpulse sehr kurz sind.

Aus der DE-A 2 843 724 ist ein Elektrotherapie- und Diagnostikgerät mit digitaler Spitzenstromanzeige bekannt, bei dem der Spitzenwert des Stromes unabhängig von dessen Kurvenform digital angezeigt wird. Dies geschieht über eine vorwärts und rückwärts zählende digitale Zähleinrichtung, einen Dekoder und eine Anzeigeeinheit durch Auswertung einer Treppenspannung, die als Steuersignal für den Strom verwendet wird. Demzufolge ist bei diesem bekannten Gerät weder eine mitlaufende - von der Kurvenform des Stromes abhängige - Stromwertanzeige noch eine genaue (deutliche) Anzeige des Momentanwertes und/oder Spitzenwertes eines aufgrund sich ändernden Patientenwiderstandes ändernden Ausgangsstromes möglich.

Aufgabe vorliegender Erfindung ist es, bei einem Reizstromgerät der eingangs genannten Art Mittel vorzusehen, die eine deutliche Anzeige des Spitzenstromwertes erlauben.

Die Aufgabe wird erfindungsgemäß durch die kennzeichnenden Merkmale des Patentanspruches 1 gelöst.

Gemäß der Erfindung verharrt die Anzeige bei jedem Strommaximum eine gewisse vorgebbare Zeit auf dem Maximalwert. Die Stromanzeige verläuft also in diesem Augenblick träger als zuvor und das Auge ist in der Lage, den Stromspitzenwert richtig abzulesen.

Weitere Vorteile und Einzelheiten der Erfindung ergeben sich aus der nachfolgenden Beschreibung eines Ausführungsbeispiels anhand der Zeichnung und in Verbindung mit den Unteransprüchen.

Es zeigen:
Figur 1 das erfindungsgemäße Reizstromgerät im Prinzipschaltbild,
Figur 2 ein Diagramm des zeitlichen Verlaufes einer Reizstromimpulsanzeige gemäß der vorliegenden Erfindung.

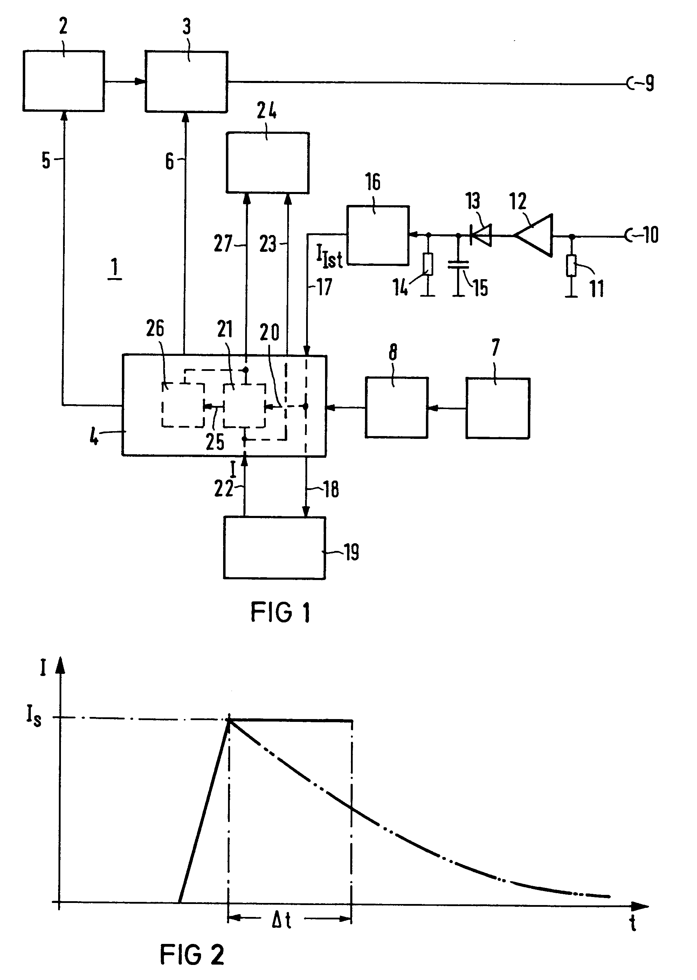

In der Figur 1 umfaßt das Reizstromgerät 1 unter anderem einen Reizstromimpulserzeuger 2 mit Intensitätseinstelleinrichtung 3, die von einem Mikroprozessor 4 über Leitungen 5 und 6 gesteuert werden. Der Intensitätseinstelleinrichtung 3 ist dabei über den Mikroprozessor 4 ein Intensitätseinstellglied 7 mit nachgeschaltetem Analog-Digital-Wandler 8 zugeordnet. Die Buchsen 9, 10 sind die Geräteanschlußbuchsen für die (nicht dargestellten) Reizstromelektroden.

Zur Messung der Istwerte I_{Ist} des Stromes beinhaltet das Reizstromgerät 1 ferner einen Meßwiderstand 11, dem über einen Verstärker 12 sowie einer Diode 13 und einem RC-Glied 14, 15 ein Analog-Digital-Wandler 16 nachgeschaltet ist. Der Analog-Digital-Wandler 16 tastet, gesteuert durch den Mikroprozessor 4, den über dem RC-Glied 14, 15 anfallenden stromproportionalen Spannungsabfall ca. 20 mal pro Sekunde ab (Istwerte des Stromes).

Jeder über die Leitung 17 erfaßte Istwert I_{Ist} wird vom Mikroprozessor 4 einerseits über eine Leitung 18 in ein RAM 19 eingespeichert und andererseits über eine Leitung 20 einer Vergleichereinheit 21 des Mikroprozessors 4 zugeleitet. Darüber hinaus ruft der Mikroprozessor 4 kontinuierlich den zuvor im RAM 19 eingespeicherten Stromwert I vom RAM 19 über eine Leitung 22 ab und führt diesen einerseits der Vergleichereinheit 21 und andererseits über eine Leitung 23 einer Stromanzeigeeinrichtung 24 zur Anzeige zu.

In der Vergleichereinheit 21 vergleicht der Mikroprozessor 4 den jeweils vorhergehend im RAM 19 abgespeicherten Stromwert I mit dem gerade angefallenen Istwert I_{Ist}. Ist der neue Istwert I_{Ist} erstmals kleiner als der vorausgegangene Stromwert I, so erkennt der Mikroprozessor 4 in der Vergleichereinheit 21 im vorhergehend abgespeicherten Stromwert einen Stromspitzenwert I_{S}. Der Mikroprozessor 4 startet daraufhin über die Leitung 25 eine innere Zähleinheit 26, die über die Leitung 27 die Stromanzeigeeinrichtung 24 in dem Sinne steuert, daß diese für eine vorgebbare Zeitdauer Δt (z.B. 0,8 s) die Stromanzeige auf dem erfaßten Stromspitzenwert I_{S} hält. Erst nach Ablauf dieser Zeitdauer zeigt die Stromanzeigeeinrichtung dann wieder den Istwert des Stromes an.

Die Figur 2 zeigt den erwähnten Vorgang anhand eines Diagramms I(t). Diese Art der Anzeige erlaubt also ein rasches und sicheres Erfassen des Spitzenstromes, was insbesondere bei Konstantspannungsbetrieb von Bedeutung ist.

## Patentansprüche

1. Reizstromgerät, insbesondere für Reizstromtherapie an einem Patienten, mit einem Reizstromimpulserzeuger und einer Stromabtasteinrichtung für Istwerte des Stromes sowie einer Stromanzeigeeinrichtung für die Anzeige der abgetasteten Istwerte des Stromes, **dadurch gekennzeichnet,** daß der Stromabtasteinrichtung (11 bis 16) eine Einrichtung (4, 19, 21) zum Erfassen des Spitzenstromes (I_{S}) nachgeschaltet ist, die eine Zeiteinheit (26) steuert, die die Anzeige des Spitzenstromwertes an der Stromanzeigeeinrichtung (24) für eine vorgebbare Zeitdauer (Δt) nach Auftreten eines Spitzenstromwertes konstant hält, wobei die Einrichtung zum Erfassen des Spitzenstromes (I_{S}) eine Vergleichereinheit umfaßt, die einen vorhergehend in einem Speicher (19) abgespeicherten Stromwert mit dem gerade angefallenen Stromistwert vergleicht und ein Konstanthaltesignal für die Spitzenwertanzeige der Stromanzeigeeinrichtung (24) erzeugt, wenn der neue Istwert (I_{Ist}) erstmals kleiner als der vorausgegangene gespeicherte Stromwert (I) ist.

2. Reizstromgerät nach Anspruch 1, **dadurch gekennzeichnet,** daß die Zeiteinheit (26) eine Zählereinheit in einem Mikroprozessor (4) ist.

3. Reizstromgerät nach Anspruch 1 oder 2, **dadurch gekennzeichnet,** daß die Vergleichereinheit (21) und der Speicher (19), z.B. RAM, Bestandteile eines Mikroprozessors (4) sind.

## Claims

1. Stimulus-current apparatus, in particular for stimulus-current therapy on a patient, having a stimulus-current impulse generator and a current-sampling device for actual values of the current and a current display device for the display of the sampled actual values of the current, characterized in that after the current-sampling device (11 to 16) there is connected a device (4, 19, 21) for the detection of the peak current (I_{S}) which device (4, 19, 21) controls a time unit (26) which keeps the display of the peak current value at the current display device (24) constant for a specifiable duration (Δt) after the occurrence of a peak current value, whereby the device for detecting the peak current (I_{S}) comprises a comparator unit which compares a current value previously stored in a memory (19) with the actual current value which has just resulted and generates a constant-holding signal for the peak value display of the current display device (24) if the new actual value (I_{Ist}) is first of all smaller than the preceding stored current value (I).

2. Stimulus-current apparatus according to claim 1, characterized in that the time unit (26) is a counter unit in a microprocessor (4).

3. Stimulus-current apparatus according to claim 1 or 2, characterized in that the comparator unit (21) and the memory (19), for example RAM, are components of a microprocessor (4).

## Revendications

1. Stimulateur, notamment pour la thérapeutique par stimulation de courant sur un patient, comportant un générateur d'impulsions de courant de stimulation et un dispositif d'échantillonnage du courant pour des valeurs instantanées du courant, ainsi qu'un dispositif d'affichage du courant pour l'affichage des valeurs instantanées et échantillonnées du courant, caractérisé en ce qu'en aval du dispositif d'échantillonnage du courant (11 à 16) est prévu un dispositif (4, 19, 21) de détection du courant de crête (I_{S}), qui commande une unité de temporisation (26), qui, après l'apparition d'une valeur de courant de crête, maintient constante, pour une durée (Δt) pouvant être déterminée à l'avance, l'affichage de la valeur de courant de crête dans le dispositif d'affichage de courant (24), le dispositif de détection du courant de crête (I_{S}) comprenant un comparateur, qui compare une valeur de courant précédemment mémorisé dans une mémoire (19) à la valeur actuelle du courant instantané et qui génère un signal constant de maintien pour l'affichage de la valeur de crête du dispositif (24) d'affichage du courant, lorsque la nouvelle valeur instantanée (I_{Ist}) est pour la première fois inférieure à la valeur précédente de courant (I) qui a été mémorisée.

2. Stimulateur selon la revendication 1, caractérisé en ce que le dispositif temporisation (26) est une unité de comptage contenue dans un microprocesseur (4).

3. Stimulateur selon la revendication 1, caractérisé en ce que le comparateur (21) et la mémoire (19), sont constitués, par exemple, par une RAM, ou par des éléments constitutifs d'un microprocesseur (4).
